# FASCICULE DE BREVET EUROPEEN

(11) **EP 1 932 570 B1**
(45) Date de publication et mention de la délivrance du brevet: **27.07.2011**
(21) Numéro de dépôt: 07023326.7
(22) Date de dépôt: 03.12.2007
(51) Int. Cl.: A63C 9/088, A61B 5/11, G01L 5/00

(54) **Procédé de contrôle de la liaison entre un individu et son engin de glisse ou de roulage et dispositif pour la mise en oeuvre du procédé**
Verfahren zur Kontrolle der Verbindung zwischen einem Individuum und seiner Gleit- oder Walzmaschine für die Umsetzung des Verfahrens
Method for controlling the attachment between a person and a gliding or rolling device and device for implementing the method

(30) Priorité: 11.12.2006 FR 0610784
(43) Date de publication de la demande: 18.06.2008
(73) Titulaire: SALOMON S.A.S., 74370 Metz-Tessy (FR)
(72) Inventeur: Damiani, Laurent, 74370 Villaz (FR); Merino, Jean-Francois, 74330 Epagny (FR); Desarmaux, Pierre, 74570 Evires (FR)

(56) Documents cités:
- WO-A-2005/018453
- FR-A1- 2 767 266
- US-A- 3 644 919
- US-A- 3 909 028
- US-A- 4 371 188
- US-A- 4 804 001
- US-A- 5 295 704

## Description

L'invention concerne un procédé de contrôle de la liaison entre un individu et son engin de glisse ou de roulage. Elle concerne également un dispositif de contrôle pour la mise en oeuvre du procédé.

Dans le domaine du ski notamment il est connu de retenir chacune des chaussures du skieur sur les skis au moyen d'éléments de retenue. Ces éléments sont usuellement de type libérable, c'est-à-dire qu'ils sont aptes à libérer la chaussure en cas de sollicitation excessive afin d'éviter dans la mesure du possible la lésion ou la fracture d'un membre.

Il existe sur le marché un grand nombre de modèles d'élément de retenue. Ces éléments sont essentiellement mécaniques c'est-à-dire que la libération de la chaussure intervient à la suite de l'ouverture d'une mâchoire de retenue de la chaussure. Cette mâchoire est rappelée par un ressort, et l'ouverture de la mâchoire intervient lorsque les efforts exercés par la chaussure sur la mâchoire surmontent l'effort de rappel exercé par le ressort.

Ces dispositifs donnent de bons résultats pour des chutes dites simples, c'est-à-dire principalement chute avant et chute en torsion. Pour les chutes dites combinées, par exemple chute avant torsion, ou chute avec vrillage, les frottements entre la chaussure et les éléments de retenue sont difficiles à maîtriser. Il existe cependant des dispositifs de compensation pour les chutes combinées. Par exemple on connaît les dispositifs qui sont décrits dans les demandes de brevet FR 2 523 857, FR 2 314 742 ou encore FR 2 707 514.

Il existe également des constructions où les efforts entre la chaussure et le ski sont captés par des jauges électroniques et sont traités par un circuit électronique qui commande la libération de la chaussure. Par exemple, on connaît le dispositif décrit dans la demande de brevet FR 2 459 669, FR 2 351 678 ou encore US 4 371 188. Le captage électronique des efforts est plus précis et moins sensible aux frottements qu'un captage mécanique. Toutefois il n'est pas totalement satisfaisant. En effet le captage des efforts au niveau de la chaussure ne permet pas de mettre en évidence certaines postures du skieur dans lesquelles il encourre un risque de lésion. De plus les chaussures actuellement sur le marché sont de plus en plus performantes, elles assurent une protection plus efficace de la partie basse de la jambe, ce qui a pour effet que les lésions en cas de chute remontent le long de la jambe et touchent davantage le genou, en particulier les ligaments du genou.

Certaines constructions ont été proposées pour réaliser un captage d'effort le long de la jambe, par exemple dans les publications de brevet US 3 909 028, ou encore FR 2 767 266. Les solutions décrites dans ces deux documents sont essentiellement mécaniques, et sont de ce fait très contraignantes pour le skieur.

Le brevet US 6 007 086 propose quant à lui de disposer des systèmes de capteurs émetteurs directionnels et de capteurs récepteurs sur les skis et sur le skieur, et de commander la libération de la chaussure lorsqu'un capteur émetteur émet son signal dans une direction en dehors du champ de réception du capteur récepteur. Un tel dispositif ne permet pas de capter avec suffisamment de précision les efforts que les membres inférieurs du skieur subissent.

Une autre solution est décrite dans le brevet US 5 295 704. Le skieur est équipé d'une sorte de genouillère et on mesure sur cet élément l'angle de flexion du membre inférieur. Cette mesure toutefois n'est pas suffisante à elle seule pour identifier les situations où le skieur s'expose à un risque de lésion.

Compte tenu de cet état de la technique, il existe un besoin pour un procédé de contrôle de la liaison entre un individu et sa ou ses planches de glisse ou de roulage qui permet de déterminer avec plus de précision les situations critiques dans lesquelles le skieur s'expose à un risque de lésion.

Ce but et d'autres buts et avantages qui apparaîtront dans la suite sont atteints par le procédé et le dispositif de l'invention.

Le procédé de contrôle de la liaison d'un individu à son engin de glisse ou de roulage auquel l'individu est retenu par un système de retenue de type libérable équipé d'un organe de retenue à deux états, un état de retenue et un état de libération est caractérisé par le fait que dans l'état de retenue de l'organe de retenue, on prend la mesure d'au moins deux angles distincts de flexion ou torsion entre la cuisse et la jambe du skieur, qu'on compare la valeur de chacun de ces angles à des valeurs indicatives de posture, et qu'on commande le passage de l'organe de retenue à l'état de libération si pour chacun des deux angles la valeur d'angle mesurée dépasse sa valeur indicative de posture.

De préférence dans le procédé de contrôle selon l'invention, on mesure l'angle correspondant à la flexion du genou (AFX) et au moins l'un des angles parmi les angles correspondants à l'inclinaison valgus (AVG), l'inclinaison varus (AVR), la rotation interne (ARI), la rotation externe (ARE).

Le dispositif de contrôle est caractérisé par le fait qu'il comprend un organe de mesure d'au moins deux angles distincts de flexion ou torsion entre la cuisse et la jambe du skieur.

De préférence dans le dispositif de contrôle selon l'invention, ledit organe de mesure mesure l'angle correspondant à la flexion du genou (AFX) et au moins l'un des angles parmi les angles correspondants à l'inclinaison valgus (AVG), l'inclinaison varus (AVR), la rotation interne (ARI), la rotation externe (ARE).

L'invention repose sur l'observation et l'identification de différentes postures critiques dans lesquelles le skieur a un risque de lésion élevé au niveau de ses genoux, en particulier au niveau des différents ligaments. Ces postures peuvent être identifiées par la prise d'au moins deux mesures d'angle au niveau de chacun des membres inférieurs du skieur.

Le procédé et le dispositif de contrôle selon l'invention peuvent être implémentés sur tout type de système de retenue libérable. Par exemple, on pourra compléter un système de retenue libérable connu et largement répandu actuellement sur le marché par le dispositif de contrôle de l'invention. Dans ce cas la butée avant et la talonnière arrière déclencheront en fonction des efforts auxquels la jambe de l'utilisateur est soumise alors qu'un déclenchement supplémentaire interviendra conformément à l'invention en fonction de la posture de l'utilisateur.

L'invention sera mieux comprise en se référant à la description ci-dessous et aux dessins en annexe qui lui sont attachés.
La figure 1 représente un skieur.
La figure 2 illustre une situation de flexion.
Les figures 3 et 4 illustrent de façon schématique des situations dites de valgus / varus.
Les figures 5 et 6 illustrent de façon schématique des situations de rotation externe / interne.
La figure 7 représente un organigramme expliquant le mode de détermination de la commande de libération.
La figure 8 montre un schéma de principe du dispositif de contrôle.

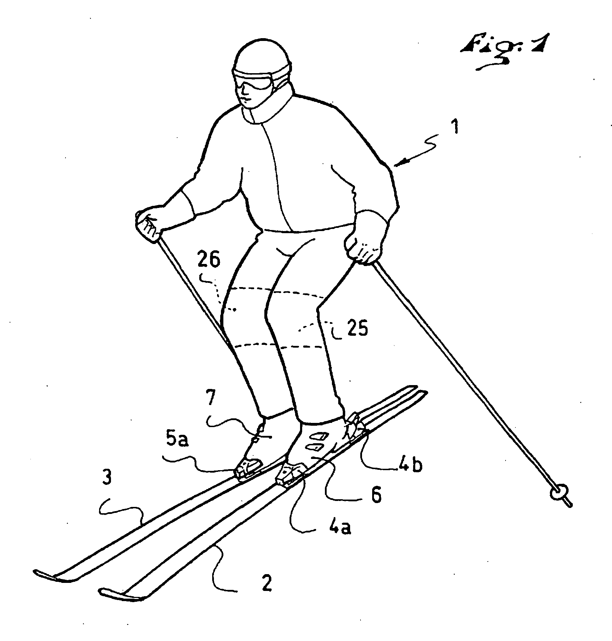

La figure 1 représente un individu 1 pratiquant la glisse sur un engin de glisse qui consiste en deux skis 2 et 3. Un système de liaison relie le skieur à son engin de glisse. Il consiste en des éléments de retenue, notamment 4a, 4b, 5a, qui retiennent chacune des chaussures 6, 7 du skieur en appui respectivement sur un ski. Ces éléments sont de type libérable, c'est-à-dire qu'ils sont aptes à libérer la chaussure en cas de sollicitation excessive. Par exemple ces éléments sont construits conformément aux demandes de brevet FR 2 843 037 et FR 2 860 729. Toutefois ces constructions ne sont pas limitatives, et toute autre construction appropriée peut également convenir. De façon connue les éléments de retenue présentent deux états principaux, un état de retenue où ils maintiennent les chaussures en appui sur le ski, et un état de libération où ils relâchent la chaussure. Dans le cas où le système de retenue comprend des éléments avant et arrière, il suffit qu'un seul de ces éléments ait une commande de passage à l'état de libération. Pour d'autres constructions connues la chaussure est retenue par un élément de retenue central.

Un dispositif de contrôle est prévu pour contrôler la liaison entre l'individu et chacun de ses skis, en particulier ce dispositif commande le passage de la liaison entre la chaussure et le ski dans son état de libération.

Dans l'état de retenue du système de retenue, le dispositif de contrôle capte les sollicitations supportées par le skieur, les analyse et délivre une commande de libération au système de liaison au cas où l'analyse des sollicitations conduit à l'identification d'une situation critique nécessitant la libération de la liaison entre le skieur et son engin, c'est-à-dire au moins l'un de ses skis dans le cas présent.

Le procédé et le dispositif de contrôle de l'invention sont focalisés sur les efforts supportés par les genoux du skieur, et ils reposent sur le concept général qu'il est nécessaire à ce niveau de mesurer au moins deux angles pour détecter certains mouvements ou certaines postures du skieur susceptibles de causer des lésions du genou.

Les mouvements observés sont les mouvements de flexion de la cuisse sur la jambe, les mouvements de varus / valgus, c'est-à-dire une déviation de la jambe par rapport à la cuisse en dedans ou en dehors, et un mouvement de rotation vers l'extérieur ou l'intérieur de la jambe relativement à la cuisse.

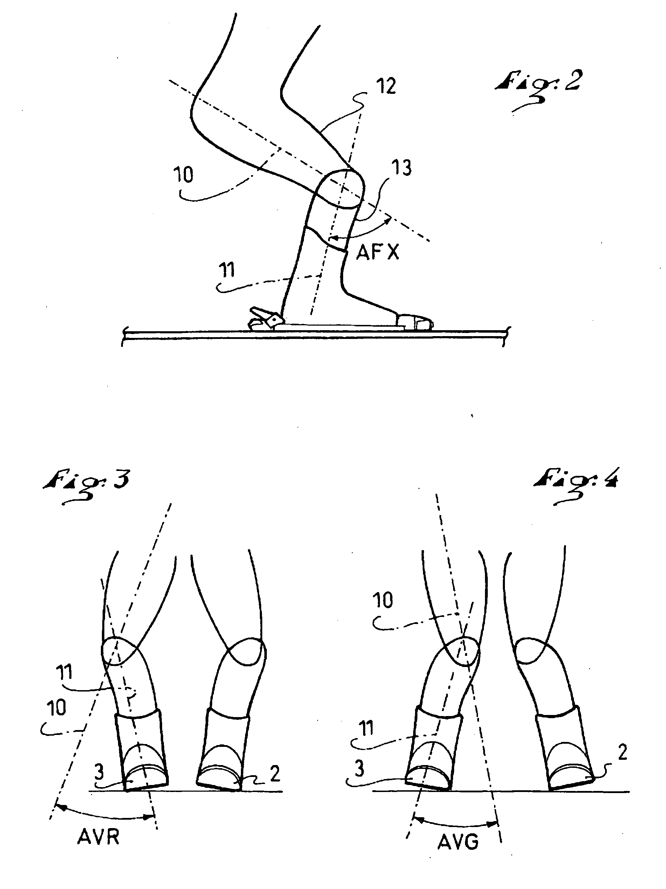

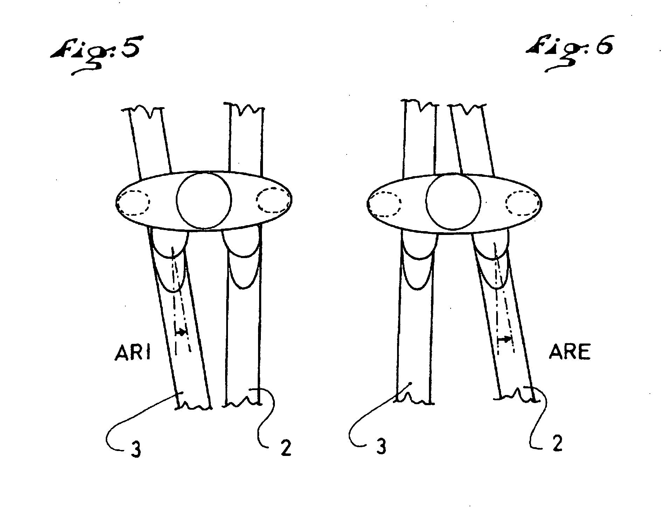

Pour observer ces mouvements, on mesure des angles au niveau de chacun des genoux. Les angles mesurés sont l'angle de flexion (AFX), cet angle est mis en évidence dans la figure 2 entre les axes 10 et 11 de la cuisse 12 et de la jambe 13, l'angle de valgus / varus (AVR, AVG), ces angles sont mis en évidence dans les figures 3 et 4, et l'angle de rotation interne / externe (ARI, ARE), ces angles sont mis en évidence dans les figures 5 et 6.

La mesure de ces angles est effectuée par tout moyen approprié. Par exemple, comme cela est suggéré dans la figure 1, on peut équiper le skieur de genouillères 25, 26. Les genouillères sont portées directement par le skieur, ou elles sont incorporées à son habillement. Les genouillères sont instrumentées, par exemple elles sont équipées de fibres conductrices qui sont intégrées dans le tissu de la genouillère en différents emplacements et selon différentes orientations et qui travaillent comme des jauges d'extensiométrie. Une autre possibilité est d'équiper la genouillère d'une centrale inertielle comprenant des goniomètres et/ou des inclinomètres. D'autres moyens de mesure peuvent également convenir comme des potentiomètres.

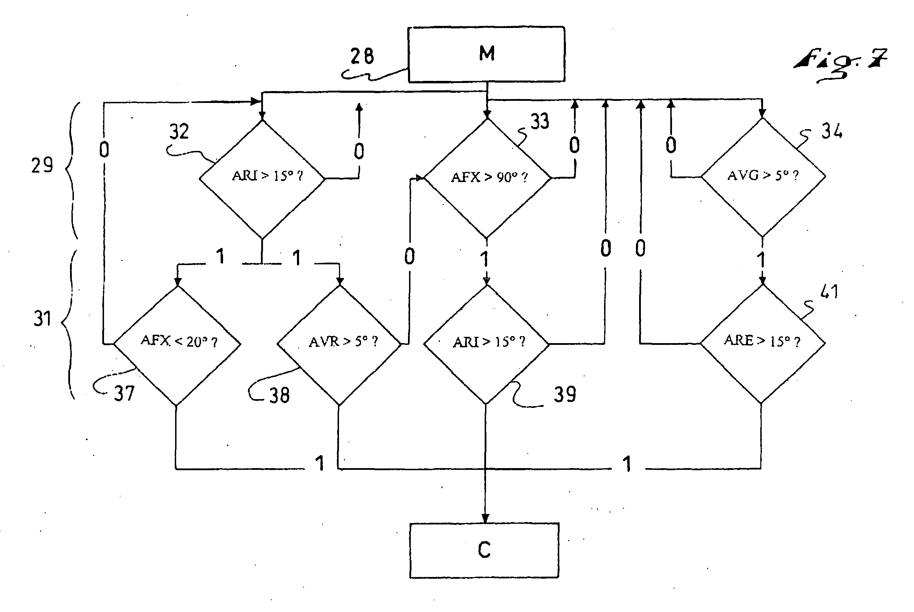

La figure 7 représente sous forme d'organigramme l'algorithme sur la base duquel une commande de passage à l'état de libération est générée. Dans l'organigramme, la commande de passage à l'état de libération est notée "C" et se trouve dans la dernière case de l'organigramme.

A chaque étape de l'organigramme, lorsque la réponse à la question posée dans le losange est "OUI", on note "1" sur la flèche reliant ce losange au losange suivant et "0" lorsque la réponse est "NON".

Selon le mode de réalisation illustré l'algorithme repose sur la mesure en temps réel des angles AFX, AVR, AVG, ARI, ARE, ce qui est exprimé dans la case 28 et noté "M".

Il est construit sur deux niveaux 29 et 31. Sur un premier niveau 29, on mesure les angles ARI, AFX et AVG, et on compare les valeurs mesurées à des valeurs indicatives, 15 degrés pour ARI, 90 degrés pour AFX, 5 degrés pour AVG. Ceci est exprimé dans les trois losanges 32, 33, 34 de l'organigramme.

Si l'un des trois angles dépasse la valeur indicative qui lui est associée, on passe au deuxième niveau 31 d'analyse.

Si c'est la valeur ARI qui a dépassé sa valeur indicative, on considère la mesure de AFX et AVR, en particulier on considère si AFX est inférieur à la valeur indicative de 20 degrés ou si AVR est supérieur à la valeur indicative de 5 degrés comme cela est exprimé dans les losanges 37 et 38. Si l'une de ces conditions est avérée, une commande de passage à l'état de libération est envoyée au système de retenue pour libérer la liaison entre l'individu et son engin de glisse.

Dans le cas où la valeur indicative de AFX est dépassée, on considère la valeur de l'angle ARI, ce qui est schématisé par le losange 39. Si ARI est supérieur à la valeur indicative de 15 degrés, une commande de libération est envoyée au système de retenue.

Si AVG a dépassé sa valeur indicative, on considère la valeur de l'angle ARE, comme le montre le losange 41. Si ARE est supérieur à la valeur indicative de 15 degrés, on adresse au système de retenue une commande de libération.

Ainsi, une commande de passage à l'état de libération est générée dans chacun des cas de figure à partir de la mesure de deux angles parmi les angles que sont l'angle de flexion, l'angle de valgus / varus, l'angle de rotation interne / externe, et la comparaison de ces angles à des valeurs indicatives de posture qui leur sont associées respectivement.

L'angle mesuré peut être l'angle réel, c'est-à-dire mesuré entre les directions définies par l'axe de la cuisse et l'axe de la jambe. Ou bien il peut être une mesure de la variation de cet angle depuis une valeur d'origine. En effet, pour le valgus / varus, et la rotation interne / externe il est connu que d'un individu à l'autre l'angle de déviation ou de rotation au repos peut varier de quelques degrés vers l'intérieur ou vers l'extérieur. Dans ce cas on peut procéder dans une phase d'initialisation à la mesure de ces angles au repos ou d'une partie d'entre eux pour ne considérer dans la suite que la variation des angles en question.

La mesure des angles peut être traitée sous toute forme appropriée, sous une forme analogique ou numérique. A et effet, la mesure peut être réalisée sous une forme continue, ou de façon séquentielle.

Chaque association d'angles indicatifs identifie une posture critique du skieur dans laquelle on estime qu'il encourre un risque de lésion au niveau du genou. Les valeurs des angles indicatifs ne sont pas des valeurs restrictives, il faut considérer ces valeurs avec une marge d'environ ±30%. D'un individu à l'autre ou d'une catégorie d'individus à l'autre il est possible que ces valeurs ne soient pas exactement les mêmes. Par exemple pour des skieurs confirmés ou des skieurs débutants les valeurs indicatives peuvent être plus ou moins élevées.

L'algorithme de la figure 7 illustre quatre postures critiques. Ce nombre n'est pas limitatif. On pourrait en considérer éventuellement moins, ou le cas échéant davantage, l'idée générale étant qu'il faut au moins deux mesures d'angles entre la cuisse et la jambe pour identifier une posture critique. Egalement on pourrait ajouter un troisième niveau au-delà du niveau 31, c'est-à-dire ajouter la mesure d'un troisième angle et sa comparaison avec un angle indicatif associé pour analyser la posture du skieur et identifier le caractère critique de la posture du skieur.

D'autre part, l'invention a pour but de prévenir seulement certains risques de lésion. C'est-à-dire qu'il est possible de piloter la commande de libération en associant la mesure et l'analyse d'autres angles ou efforts, par exemple des angles ou efforts au niveau de la cheville et/ou de la chaussure de l'individu.

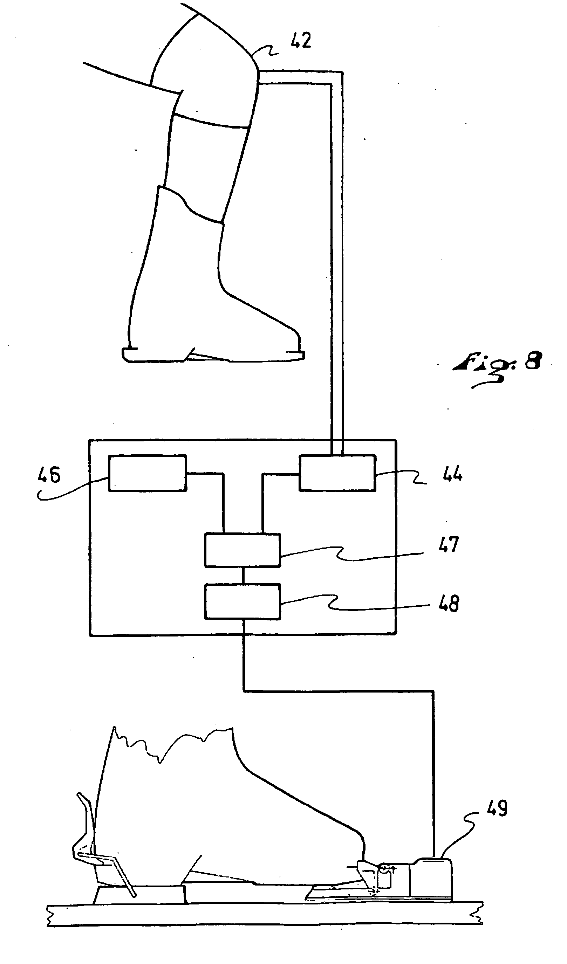

La figure 8 montre un schéma de principe du dispositif de contrôle. Elle représente un organe de mesure sous la forme d'une genouillère 42. Cet organe est apte à mesurer au moins deux angles de flexion ou rotation du membre inférieur du skieur parmi les angles que sont l'angle de flexion, l'angle de valgus / varus, l'angle de rotation interne / externe. D'autres constructions peuvent aussi convenir pour cet organe de mesure. Le signal issu de l'organe de mesure est envoyé à un circuit 44 de mise en forme du signal.

Le dispositif comporte par ailleurs une table 46 qui contient les valeurs indicatives de posture associées à chacun des angles mesurés. Un circuit de traitement 47 analyse les angles mesurés et les considère relativement aux valeurs indicatives, conformément à ce qui a été décrit précédemment.

Le circuit de traitement 47 pilote un circuit de commande 48 qui est apte à envoyer à une commande de passage à l'état de libération au système de retenue. Par exemple comme cela est représenté, le circuit 48 pilote l'ouverture d'un élément de retenue avant 49 conforme à la demande de brevet FR 2 843 037 déjà citée. D'autres constructions de systèmes de retenue conviennent également.

La transmission entre les différents composants du dispositif est réalisée par tout moyen approprié filaire ou non filaire. Egalement les signaux peuvent être de nature analogique ou numérique.

Naturellement la présente description n'est donnée qu'à titre indicatif, et l'on pourrait adopter d'autres mises en oeuvre de l'invention sans pour autant sortir du cadre de celle-ci.

En particulier l'invention n'est pas limitée au domaine du ski, elle est applicable à toute pratique de la glisse ou du roulage où l'individu est retenu sur son engin par au moins un élément de retenue de type libérable, notamment le surf de neige, le ski de télémark, le ski de fond, et autre encore. Selon la pratique du sport et selon la catégorie d'individus en question les valeurs indicatives peuvent être différentes de celles qui ont été indiquées.

## Revendications

1. Procédé de contrôle de la liaison d'un individu (1) à son engin de glisse ou de roulage (2, 3) auquel l'individu est retenu par un système de retenue (4a, 4b, 5a) de type libérable équipé d'un organe de retenue à deux états, un état de retenue et un état de libération est **caractérisé par le fait que** dans l'état de retenue de l'organe de retenue, on prend la mesure d'au moins deux angles distincts de flexion ou torsion entre la cuisse et la jambe du skieur (AFX, AVR, AVG, ARI, ARE), qu'on compare la valeur de chacun de ces angles à une valeur indicative de posture, et qu'on commande le passage de l'organe de retenue à l'état de libération lorsque pour chacun des angles la valeur d'angle mesurée dépasse sa valeur indicative.

2. Procédé de contrôle selon la revendication 1, **caractérisé en ce qu'**on prend la mesure de l'angle correspondant à la flexion du genou (AFX) et au moins l'un des angles parmi les angles correspondants à l'inclinaison valgus (AVG), l'inclinaison varus (AVR), la rotation interne (ARI), la rotation externe (ARE).

3. Procédé selon la revendication 1, **caractérisé par le fait qu'**on effectue la mesure d'au moins deux angles parmi les angles de flexion, de varus, de valgus, de rotation interne, de rotation externe (AFX, AVR, AVG, ARI, ARE) sur deux niveaux (29,31).

4. Procédé selon la revendication 3, **caractérisé par le fait qu'**on considère sur un premier niveau la mesure d'au moins un angle parmi l'angle de rotation interne (ARI), l'angle de flexion (AFX), l'angle de valgus (AVG).

5. Procédé selon la revendication 4, **caractérisé par le fait qu'**on mesure l'angle de rotation interne (ARI), et l'angle de flexion (AFX), et qu'on considère si l'angle de rotation interne (ARI) est supérieur à la valeur indicative de 15 degrés environ et l'angle de flexion inférieur à la valeur indicative de 20 degrés environ.

6. Procédé selon la revendication 4, **caractérisé par le fait que** l'on mesure l'angle de rotation interne (ARI) et l'angle de varus, et qu'on considère si l'angle de rotation interne (ARI) est supérieur à la valeur indicative de environ 15 degrés environ et l'angle de varus supérieur à la valeur indicative de 5 degrés environ.

7. Procédé selon la revendication 4, **caractérisé par le fait qu'**on mesure l'angle de flexion (AFX) et l'angle de rotation interne (ARI), et qu'on considère si l'angle de flexion (AFX) est supérieur à la valeur indicative de 90 degrés environ et l'angle de rotation interne supérieur à la valeur indicative de 15 degrés environ.

8. Procédé selon la revendication 4, **caractérisé par le fait qu'**on mesure l'angle de valgus (AVG) et l'angle de rotation externe (ARE) et qu'on considère si l'angle de valgus (AVG) est supérieur à la valeur indicative de 5 degrés environ et l'angle de rotation externe (ARE) supérieur à la valeur indicative de 15 degrés environ.

9. Procédé selon l'une des revendications précédentes, **caractérisé par le fait qu'**on procède dans une phase d'initialisation à la mesure d'au moins une partie des angles au repos.

10. Dispositif de contrôle de la liaison d'un individu (1) à son engin de glisse ou de roulage (2, 3) auquel l'individu est retenu par un système de retenue (4a, 4b, 5a) de type libérable équipé d'un organe de retenue à deux états, un état de retenue et un état de libération, **caractérisé par le fait qu'**il comprend un organe de mesure (42) d'au moins deux angles distincts de flexion ou torsion entre la cuisse et la jambe du skieur (AFX, AVR, AVG, ARE, ARI).

11. Dispositif selon la revendication 10, **caractérisé en ce que** ledit organe de mesure (42) mesure l'angle correspondant à la flexion du genou (AFX) et au moins l'un des angles parmi les angles correspondants à l'inclinaison valgus (AVG), l'inclinaison varus (AVR), la rotation interne (ARI), la rotation externe (ARE).

12. Dispositif selon la revendication 11, **caractérisé par le fait qu'**il comprend une table (46) de valeurs indicatives associées à au moins une partie des angles de flexion ou torsion (AFX, AVR, AVG, ARE, ARI).

13. Dispositif selon la revendication 11, **caractérisé par le fait qu'**il comprend un circuit (47) de traitement qui analyse les signaux de mesure issus de l'organe de mesure (42) et les considère relativement aux valeurs indicatives contenues dans la table (46).

## Claims

1. Method for controlling the connection between an individual (1) and his gliding or rolling device (2, 3), on which the individual is retained by a retaining system (4a, 4b, 5a) of the releasable type, said retaining system (4a, 4b, 5a) being equipped with a retaining member having two states, a retaining state and a released state, **characterized in that** in the retaining state of the retaining member, at least two different bending or twist angles (AFX, AVR, AVG, ART, ARE) between the thigh and the shank of the skier are measured, **in that** the value of each of these angles is compared with an indicative value for posture, and **in that** the retaining member is passed into the released state when, for each of the angles, the value of the angle measured exceeds the indicative value thereof.

2. Control method according to Claim 1, **characterized in that** the angle (AFX) corresponding to the bending of the knee and at least one of the angles from among the angles corresponding to the valgus inclination (AVG) , the varus inclination (AVR), the internal rotation (ARI) and the external rotation (ARE) are measured.

3. Method according to Claim 1, **characterized in that** at least two angles from among the bending angle, the varus angle, the valgus angle, the internal rotation angle and the external rotation angle (AFX, AVR, AVG, ARI, ARE) are measured on two levels (29, 31).

4. Method according to Claim 3, **characterized in that** on a first level at least one angle from among the internal rotation angle (ARI), the bending angle (AFX) and the valgus angle (AVG) is measured.

5. Method according to Claim 4, **characterized in that** the internal rotation angle (ARI) and the bending angle (AFX) are measured and **in that** it is considered whether the internal rotation angle (ARI) is greater than the indicative value of around 15 degrees and the bending angle is less than the indicative value of around 20 degrees.

6. Method according to Claim 4, **characterized in that** the internal rotation angle (ARI) and the varus angle are measured and **in that** it is considered whether the internal rotation angle (ARI) is greater than the indicative value of around 15 degrees and the varus angle is greater than the indicative value of around 5 degrees.

7. Method according to Claim 4, **characterized in that** the bending angle (AFX) and the internal rotation angle (ARI) are measured and **in that** it is considered whether the bending angle (AFX) is greater than the indicative value of around 90 degrees and the internal rotation angle is greater than the indicative value of around 15 degrees.

8. Method according to Claim 4, **characterized in that** the valgus angle (AVG) and the external rotation angle (ARE) are measured and **in that** it is considered whether the valgus angle (AVG) is greater than the indicative value of around 5 degrees and the external rotation angle (ARE) is greater than the indicative value of around 15 degrees.

9. Method according to one of the preceding claims, **characterized in that** in an initialization phase at least some of the angles at rest are measured.

10. Device for controlling the connection between an individual (1) and his sliding or rolling device (2, 3), on which the individual is retained by a retaining system (4a, 4b, 5a) of the releasable type, said retaining system (4a, 4b, 5a) being equipped with a retaining member having two states, a retaining state and a released state, **characterized in that** said device comprises a measuring member (42) for measuring at least two different bending or twist angles (AFX, AVR, AVG, ARE, ARI) between the thigh and the shank of the skier.

11. Device according to Claim 10, **characterized in that** said measuring member (42) measures the angle corresponding to the bending of the knee (AFX) and at least one of the angles from among the angles corresponding to the valgus inclination (AVG), the varus inclination (AVR), the internal rotation (ART) and the external rotation (ARE).

12. Device according to Claim 11, **characterized in that** it comprises a table (46) of indicative values associated with at least some of the bending or twist angles (AFX, AVR, AVG, ARE, ARI).

13. Device according to Claim 11, **characterized in that** it comprises a processing circuit (47) which analyses the measurement signals from the measuring member (42) and considers them in relation to the indicative values contained in the table (46).

## Patentansprüche

1. Verfahren zur Kontrolle der Verbindung einer Person (1) mit ihrem Gleit- oder Rollgerät (2, 3), an dem die Person durch ein freisetzbares Haltesystem (4a, 4b, 5a) gehalten ist, das mit einem Halteelement mit zwei Zuständen versehen ist, und zwar einem Haltezustand und einem Freisetzungszustand, **dadurch gekennzeichnet, dass** im Haltezustand des Halteelements mindestens zwei bestimmte Flexions- oder Torsionswinkel zwischen dem Ober- und dem Unterschenkel des Skifahrers (AFX, AVR, AVG, ARI, ARE) gemessen werden, dass der Wert jedes dieser Winkel mit einem Haltungsrichtwert verglichen wird und dass der Übergang des Halteelements in den Freisetzungszustand gesteuert wird, wenn der gemessene Winkelwert für jeden der Winkel seinen Richtwert übersteigt.

2. Kontrollverfahren nach Anspruch 1, **dadurch gekennzeichnet, dass** der der Flexion des Knies entsprechende Winkel (AFX) und mindestens einer der Winkel unter den Winkeln, die der Valgusneigung (AVG), der Varusneigung (AVR), der Innenrotation (ARI) und der Außenrotation (ARE) entsprechen, gemessen werden.

3. Verfahren nach Anspruch 1, **dadurch gekennzeichnet, dass** mindestens zwei winkel unter dem Flexions-, dem Varus-, dem Valgus-, dem Innenrotations- und dem Außenrotationswinkel (AFX, AVR, AVG, ARI, ARE) auf zwei Ebenen (29, 31) gemessen werden.

4. Verfahren nach Anspruch 3, **dadurch gekennzeichnet, dass** auf einer ersten Ebene das Maß mindestens eines Winkels unter dem Innenrotationswinkel (ARI), dem Flexionswinkel (AFX) und dem Valguswinkel (AVG) bestimmt wird.

5. Verfahren nach Anspruch 4, **dadurch gekennzeichnet, dass** der Innenrotationswinkel (ARI) und der Flexionswinkel (AFX) gemessen werden und bestimmt wird, ob der Innenrotationswinkel (ARI) ungefähr 15 Grad über dem Richtwert liegt und der Flexionwinkel ungefähr 20 Grad unter dem Richtwert liegt.

6. Verfahren nach Anspruch 4, **dadurch gekennzeichnet, dass** der Tnnenrotationswinkel (ARI) und der Varuswinkel gemessen werden und bestimmt wird, ob der Innenrotationswinkel (ARI) ungefähr 15 Grad über dem Richtwert liegt und der Varuswinkel ungefähr 5 Grad über dem Richtwert liegt.

7. Verfahren nach Anspruch 4, **dadurch gekennzeichnet, dass** der Flexionswinkel (AFX) und der Innenrotationswinkel (ARI) gemessen werden und bestimmt wird, ob der Flexionswinkel (AFX) ungefähr 90 Grad über dem Richtwert liegt und der Innenrotationswinkel ungefähr 15 Grad über dem Richtwert liegt.

8. Verfahren nach Anspruch 4, **dadurch gekennzeichnet, dass** der Valguswinkel (AVG) und der Außenrotationswinkel (ARE) gemessen werden und bestimmt wird, ob der Valguswinkel (AVG) ungefähr 5 Grad über dem Richtwert liegt und der Außenrotationswinkel (ARE) ungefähr 15 Grad über dem Richtwert liegt.

9. Verfahren nach einem der vorhergehenden Ansprüche, **dadurch gekennzeichnet, dass** in einer Initialisierungsphase die Messung mindestens eines Teils der Winkel in Ruhe vorgenommen wird.

10. Vorrichtung zur Kontrolle der Verbindung einer Person (1) mit ihrem Gleit- oder Rollgerät (2, 3), an dem die Person durch ein freisetzbares Haltesystem (4a, 4b, 5a) gehalten ist, das mit einem Halteelement mit zwei Zuständen versehen ist, und zwar einem Haltezustand und einem Freisetzungszustand, **dadurch gekennzeichnet, dass** sie ein Element (42) zum Messen von mindestens zwei bestimmten Flexions- oder Torsionswinkel zwischen dem Ober- und dem Unterschenkel des Skifahrers (AFX, AVR, AVG, ARI, ARE) umfasst.

11. Vorrichtung nach Anspruch 10, **dadurch gekennzeichnet, dass** das Messelement (42) den der Flexion des Knies entsprechenden Winkel (AFX) und mindestens einen der Winkel unter den Winkeln, die der Valgusneigung (AVG), der Varusneigung (AVR), der Innenrotation (ART) und der Außenrotation (ARE) entsprechen, misst.

12. Vorrichtung nach Anspruch 11, **dadurch gekennzeichnet, dass** sie eine Tabelle (46) mit Richtwerten umfasst, die mindestens einem Teil der Flexions- oder Torsionswinkel (AFX, AVR, AVG, ARI, ARE) zugeordnet sind.

13. Vorrichtung nach Anspruch 11, **dadurch gekennzeichnet, dass** sie eine Verarbeitungsschaltung (47) umfasst, die die vom Messelement (42) gesandten Messsignale analysiert und sie unter Bezug auf die in der Tabelle (46) enthaltenen Richtwerten bestimmt.
